# EUROPEAN PATENT APPLICATION

(11) **EP 4 286 535 A1**
(43) Date of publication of application: **06.12.2023**
(21) Application number: 22176980.5
(22) Date of filing: 02.06.2022
(51) Int. Cl.: C12Q 1/6883, G01N 33/50

(54) **METHOD AND DETECTION KIT FOR THE DETECTION OF PYROGENS IN BIOLOGICAL SAMPLES**

(71) Applicant: Minerva Biolabs GmbH, 12681 Berlin (DE)
(72) Inventor: PALAND, Nicole, 13125 BERLIN (DE); VOLLENBROICH, Dirk, 12683 BERLIN (DE)
(74) Representative: Wehlan, Helmut

(57) **Abstract**

The invention relates to a method and a detection kit for the detection of pyrogens in a biological sample, in which immune cells, preferably the monocytic cell line THP-1 or THP-1 macrophages, are brought into contact with the sample, which may contain pyrogens, whereby the expression of immunomodulatory mediators takes place, wherein after lysis of the immune cells the nucleic acids of the immune cells are extracted and the expression of immunomodulatory mediators is detected by means of PCR. At least one of three different cytokines/chemokines, preferably IL-1β, TNFa or CXCL8/IL-8, are detected in parallel. The invention can be used for the detection of endotoxin and non-endotoxin pyrogenic contaminations in a medicinal sample.

## Description

### FIELD OF INVENTION

The invention generally presents an improved monocyte activation test based on nucleic acid amplification techniques (NAT-MAT). The present invention will enable the detection of pyrogens in medicinal products reliably and fast by the employment of the techniques of the polymerase chain reaction (PCR), quantitative real-time PCR (qPCR) and digital PCR (dPCR). The invention comprises the process from treating immune competent cells with the medicinal product to be tested, the extraction of nucleic acids and the detection of cytokines by PCR-techniques. The invention also includes a detection kit that was developed and can be used for the detection of endotoxin and non-endotoxin pyrogenic contaminations in a medicinal sample. The invention is set out in the claims.

### BACKGROUND OF INVENTION

An essential step in the production of pharmaceutical products is the safety testing. Parenteral-applied medicinal products and devices have to be tested for the presence of fever-causing contaminations, termed pyrogens. In general, they are of biological origin but can also be non-biological. Pyrogens can cause a systemic response when they get into the circulatory system of humans or other mammalians. This systemic response is known as the inflammatory response or inflammation in short, and is mediated by the many different cells of the immune system. It is an important defence mechanism that protects the organism from severe infection by pathogens. It combats the pathogenic invader or starts the healing process in the case of an injury. The immune defence system comprises two main mechanisms. One mechanism is he translocation of immune cells, namely leukocytes such as B- and T- lymphocytes or monocytes/macrophages to the site of infection through the blood- and lymph system. The other mechanism is the systemic rise of temperature leading to fever. Fever results in an overheating of the environment of the pathogen so that it turns hostile against it. The invader itself is responsible for the rise in temperature because its own compounds cause a fever response. As initially mentioned, these compounds are termed pyrogens or pyrogenic compound. One discriminates exogenous and endogenous pyrogens. Exogenous pyrogens are components of microbial entities like bacteria, viruses or fungus. Usually, they are found on the surface of the microbe, i.e., the endotoxin lipopolysaccharide (LPS) is found on gram-negative bacteria, lipoteichoic acid (LTA) on gram-positive bacteria or others found in viruses or fungus. Exogenous pyrogens trigger the release of endogenous pyrogens from the infiltrated immune cells to the site of infection or injury. They are released by the leukocytes, by malignoms or by tissue trauma. Endogenous pyrogens are pro-inflammatory mediators such as cytokines and chemokines, i.e., Interleukin (IL)-1, IL-6, Tumor Necrosis Factor (TNFα) or CXCL8/IL-8 to name only a few.

Apart from the role as a pyrogen, released pro-inflammatory mediators attract other immune cells to translocate to the site of infection and release further cytokines and chemokines. Sophisticated regulatory mechanisms ensure that just enough pro-inflammatory cytokines and chemokines are present to fight the invader because too many pro-inflammatory mediators can damage the organism locally but especially systemically when the invader and thus the immune response gets into the circulatory system. Likewise, a dysregulation of the immune response can lead to a local or systemic hyperinflammation. Local and systemic hyperinflammation can culminate in a life-threatening septic shock. Factors aiding the development of a sepsis depend on the type and the amount of the exogenous or endogenous pyrogen as well as on the sensitivity and the predisposition of the individual to hyperinflammation. Exogenous pyrogens can also be of non-biological origin like metal compounds in elastomers or rubber abrasion that could get into medicinal products.

For the prevention of a systemic inflammatory response culminating in hyperinflammation and a systemic shock or sepsis, the safety of pharmaceutical products that are administered parenteral that is intravenous, intramuscular or subcutaneous must be monitored closely for the presence of pyrogens. The European Pharmacopoeia describes two animal-based tests. That is the Rabbit Pyrogen Test (RPT) and the *Limulus Amoebocyte* Lysate (LAL), also known as the bacterial endotoxin test (BET). They are currently routinely used to test pharmaceutical parenteral products.

The Rabbit Pyrogen Test is described in the European Pharmacopoeia, (EP 2.6.8) as well as in the United States Pharmacopeia (USP<151>). It is an *in vivo* test where the rise of body temperature in a statistically significant number of rabbits is tested. A sterile solution of the pharmaceutical product that needs to be tested is injected intravenously and an average of the body temperature of the tested animals is calculated. The RPT is sensitive to a plethora of pyrogens including endotoxin and non-endotoxin compounds but it has a low sensitivity. It is in the range of nanograms endotoxin/ml in comparison to picogram endotoxin/ml in other pyrogen tests such as the LAL for example. Moreover, only an approximate correlation between the pyrogen and the fever reaction between rabbit species with variation up to 10,000-fold can be made. Furthermore, since the EU directive 2010/63 ("Protection of Experimental Animals"), employment of the RTP have become stricter and since 2018 5-year re-approvals are rejected. Thus, variability between rabbit species, insensitivity, inadequate quantitative results, ethical reasons and especially the timely limited approval for the test makes the RPT, at least in Europe, in the very near future obsolete.

An alternative to the RTP is the LAL. It is described in the European Pharmacopoeia (EP 2.6.14) as well as in the United States Pharmacopeia (USP<85>). It is an *ex vivo* test where the amoebocyte lysate is taken from the horseshoe crab (*Limulus polyphemus*)*.* The red blood cells in the lysate react with bacterial endotoxin in the medical product that needs to be tested by coagulation. The coagulation can be measured then. *Limulus polyphemus* is located at the east coast of the USA where the population is strictly monitored and regulated. For removing the amoebocyte lysate from the animals they need to be captured and transported to the site of blood collection. After drawing blood, they are transported back and released to their environment. It is estimated that 5 to 15% of these animals, depending on who does the estimation, do not survive. This is an important issue because *Limulus polyphemus* is listed as an endangered animal. Moreover, three other Asian horseshoe crab species are already listed as threatened to get extinct for reasons other than the LAL. Since there is a constant global rise in the performed LAL tests, the availability of the animals is decreasing. This might endanger drug safety and gives rise to a global discussion. The LAL has other limitations because it can only detect endotoxin LPS from gram-negative bacteria but not pyrogens of non-endotoxin origin (Moltz, 1993; Tilders et al., 1994; Zeisberger & Roth, 1993). Thereby, the possible potency of non-endotoxine contamination in pharmaceutical products for humans is not completely reflected with the LAL. For this reason, other tests usually the RFP are also additionally employed.

These two animal-based test systems have strong limitations in availability, sensitivity, specifity and ethical reasons. Other non-animal-based tests are needed. The requirements for those tests comprise high sensitivity, a specifity for a broad range of pyrogens and an unlimited availability. One non-animal alternative fulfilling all of these requirements is the Monocyte Activation Test. It is described in the European Pharmacopoeia (EP 2.6.30). As early as in the 1990ies models which utilizes the ability of immune cells to react to pyrogens were described as potential alternative candidates to the RPT and the LAL (A et al., 1999; Eperon et al., 1997; Hartung & Wendel, 1995). Several different methods were employed and described utilizing peripheral blood mononuclear cells (PBMCs) (Hartung & Wendel, 1995)from humans, monocytic cell lines like MonoMac 6 (MM6) (Ziegler-Heitbroc et al., 1988) or THP-1 (Auwerx, 1991; Tsuchiya et al., 1980)as well as murine macrophagic cell lines RAW264.7. THP-1 cells can be cultured and used as monocytes which are cells in suspension which can be differentiated to THP-1 derived macrophages which are metabolically inactive but more sensitive to the detection of pyrogens by an increased cluster of differentiation (CD)-14. Readouts vary from the cytokines IL-1β, IL-6 or TNF which are described in the European Pharmacopoeia EP 2.6.30 and non-pyrogenic metabolites neopterin or nitrite (Hartung et al., 2001).

The European Centre for the Validation of Alternative Methods (ECVAM) has summarized and described six different alternative pyrogen tests based on leukocyte cell lines or on human whole blood.

Pyrogen tests based on leukocyte cell lines include tests utilizing MM6, THP-1 cells (Eperon & Jungi, 1996; Peterbauer et al., 2000; TAKTAK et al., 1991; Werner-Felmayer et al., 1995).

The test based on MM6 detects IL-6 or TNF in the supernatant with commercially available ELISA kits. Sensitivity was 0.125IU/ml for Endotoxin Standard Biological Reference Preparation Batch No. 2 in opposition to 0.03IU/ml for LAL. The limit of detection for LPS is 10pg/ml. The test can be inhibited by the presence of immunoglobulin G. The MM6 test can be used in combination with the LAL for the testing of some bacterial vaccines (TAKTAK et al., 1991).

Utilizing THP-1 cells, it could be shown that the detection of pyrogens from gram-negative bacteria was in correlation to the detection limit of the LAL but superior for the detection of pyrogens from gram-positive bacteria in comparison to the LAL. Detection of pyrogens was determined by the measurement of TNF or neopterin (Peterbauer et al., 2000). The limit of detection was by 1-10 pg/ml endotoxin.

Pyrogen tests based on fresh human whole blood were developed to measure the production and secretion of the pro-inflammatory cytokines IL-1β, IL-6, TNF or prostaglandin E2 (PG2) by ELISA. These tests correlated with the amount of pyrogen that were present in the tested sample (Fennrich et al., 1999; Hartung & Wendel, 1996; Pool et al., 1998). Two tests are established with the endpoint detection of IL-1βor IL-6 with an ELISA. Detection limits lie at <50 pg/ml LPS/ml or 3pg/ml LPS for a one plate assay and 0.06IU/ml, that is 6pg/ml LPS for a two-plate assay as described in Novel Pyrogen Tests Based on the Human Fever Reaction. The Report and Recommendations of ECVAM Workshop 43 (Hartung et al., 2001).

Although several alternative methods have been described that are comparable to the LAL they are not yet widely and routinely used. Additionally, the ban on performing the Rabbit Pyrogen Testing and the threatening of extinction for the horseshoe crab *Limulus polyphemus* requires the development of reliable broadly applicable pyrogen testing test in order not to endanger drug safety.

### SUMMARY OF INVENTION

The present invention discloses an advanced *in vitro* pyrogen test based on the monocytic THP-1 cell line model of the monocyte/macrophage system. This model is directed to detect endotoxin and non-endotoxin pyrogens from medicinal products based on the requirements of the Monocyte Activation Assay but instead of detecting secreted proteins by ELISA as is described in the European Pharmacopoeia EP 2.6.30 detecting the expression of pro-inflammatory cytokines by measuring the amplification of messenger RNA (mRNA) by the aid of the polymerase chain reaction (PCR), either using the well-established quantitative real-time PCR (qPCR) or the newly established digital PCR (dPCR).

For the first time, a method was developed ranging from cultivating monocytes/macrophages to the detection of cytokines by PCR. The system functions with both monocytes or macrophages. In a first step the cells are cultivated and either differentiated to macrophages or left as monocytes. The cells are plated on a cell culture plate in 96-well scale and combined with the pharmaceutical product to be tested in three different dilutions in four replicates. After a certain incubation time, the cells in a next step are lysed and the nucleic acids are extracted. The extracts are subjected to amplification by qPCR or dPCR.

The invention comprises a primer/probe system for the detection of at least three cytokines/chemokines IL-1β, IL-6 and IL-8 simultaneously. Additionally, a fourth cytokine TNF or an internal extraction control can be added which is a synthetic RNA.

The invention has been developed to detect endotoxin pyrogens and non-endotoxin pyrogens alike.

The invention provides a kit that contains an assay system comprises the cell system, a primer/probe system for the detection of the mentioned cytokines/chemokines and a housekeeping gene to ensure data accuracy, two lyophilized mastermixes containing or lacking a reverse transcriptase for performing dPCR and an analysis program for easy analysis of the data obtained by dPCR.

Other objects and features of this invention will be described hereinafter.

### BRIEF DESCRIPTION OF THE DRAWINGS

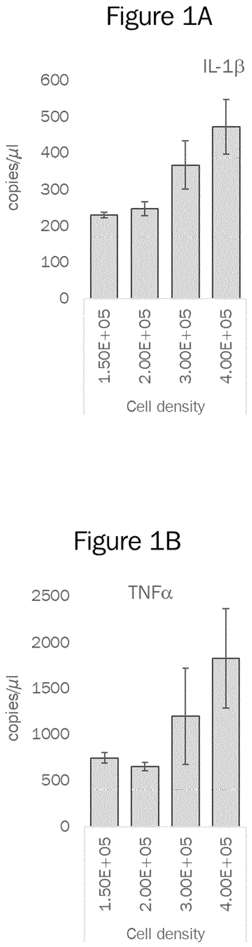

Figure 1A is a graph that represents the base line gene expression measured by digital PCR of IL-1þ in THP-1 monocytes in four different cell densities (150,000 cells per 100 µl/96-well, 200,000 100 µl/96-well, 300,000 cells 100 µl/96-well, 400,000 cells per 100 µl/96-well) in untreated control cells in a monocyte activation assay presenting the capability of the system to detect gene expression of IL-1β.
Figure 1B is a graph that represents the base line gene expression measured by digital PCR of TNFα in THP-1 monocytes in four different cell densities (150,000 cells per 100 µl/96-well, 200,000 100 µl/96-well, 300,000 cells 100 µl/96-well, 400,000 cells per 100 µl/96-well) in untreated control cells in a monocyte activation assay presenting the capability of the system to detect gene expression of IL-6.

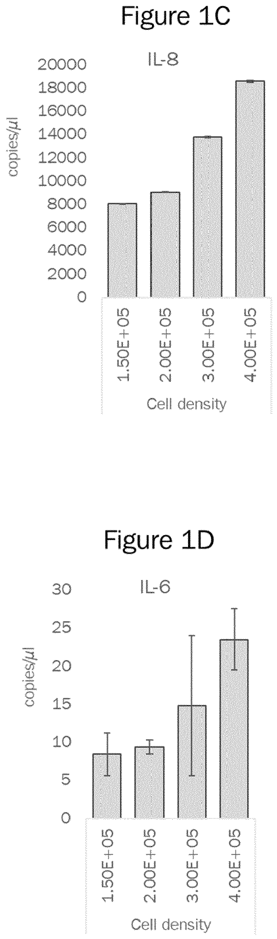

Figure 1C is a graph that represents the base line gene expression measured by digital PCR of IL-8 in THP-1 monocytes in four different cell densities (150,000 cells per 100 µl/96-well, 200,000 100 µl/96-well, 300,000 cells 100 µl/96-well, 400,000 cells per 100 µl/96-well) in untreated control cells in a monocyte activation assay presenting the capability of the system to detect gene expression of IL-8.
Figure 1D is a graph that represents the base line gene expression measured by digital PCR of IL-6 in THP-1 monocytes in four different cell densities (150,000 cells per 100 µl/96-well, 200,000 100 µl/96-well, 300,000 cells 100 µl/96-well, 400,000 cells per 100 µl/96-well) in untreated control cells in a monocyte activation assay presenting the capability of the system to detect gene expression of TNFα.

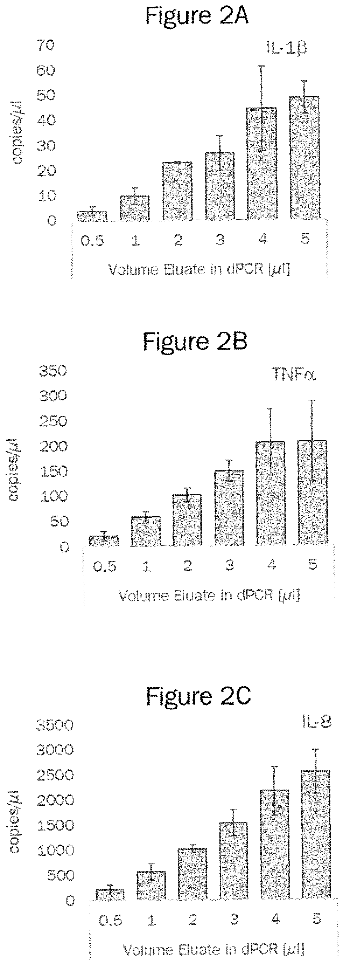

Figure 2A is a graph that represents the base line gene expression of IL-1þ when different volumes of extracts (0.5 µl in a 12 µl dPCR-reaction, 1 µl in a 12 µl dPCR-reaction, 2 µl in a 12 µl dPCR-reaction, 3 µl in a 12 µl dPCR-reaction, 4 µl in a 12 µl dPCR-reaction, 5 µl in a 12 µl dPCR-reaction) were amplified with digital PCR showing a linear increase of gene expression with increasing volumes.
Figure 2B is a graph that represents the base line gene expression of TNFα when different volumes of extracts (0.5 µl in a 12 µl dPCR-reaction, 1 µl in a 12 µl dPCR-reaction, 2 µl in a 12 µl dPCR-reaction, 3 µl in a 12 µl dPCR-reaction, 4 µl in a 12 µl dPCR-reaction, 5 µl in a 12 µl dPCR-reaction) were amplified with digital PCR showing a linear increase of gene expression with increasing volumes.
Figure 2C is a graph that represents the base line gene expression of IL-8 when different volumes of extracts (0.5 µl in a 12 µl dPCR-reaction, 1 µl in a 12 µl dPCR-reaction, 2 µl in a 12 µl dPCR-reaction, 3 µl in a 12 µl dPCR-reaction, 4 µl in a 12 µl dPCR-reaction, 5 µl in a 12 µl dPCR-reaction) were amplified with digital PCR showing a linear increase of gene expression with increasing volumes.

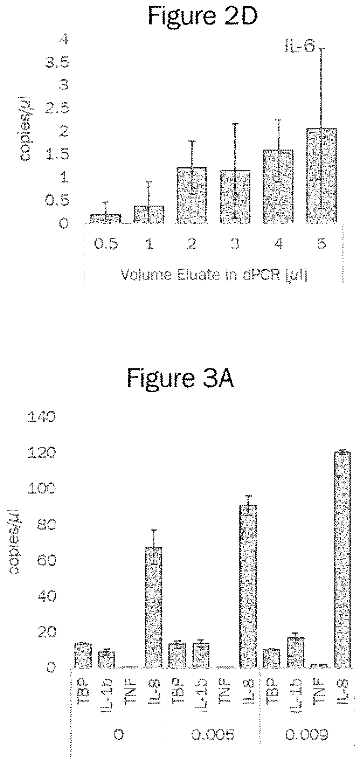

Figure 2D is a graph that represents the base line gene expression of IL-6 when different volumes of extracts (0.5 µl in a 12 µl dPCR-reaction, 1 µl in a 12 µl dPCR-reaction, 2 µl in a 12 µl dPCR-reaction, 3 µl in a 12 µl dPCR-reaction, 4 µl in a 12 µl dPCR-reaction, 5 µl in a 12 µl dPCR-reaction) were amplified with digital PCR showing a linear increase of gene expression with increasing volumes.
Figure 3A is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is to endotoxin lipopolysaccharide (LPS) from *Escherichia coli* (0, 0.005 EU/ml and 0.009 EU/ml).

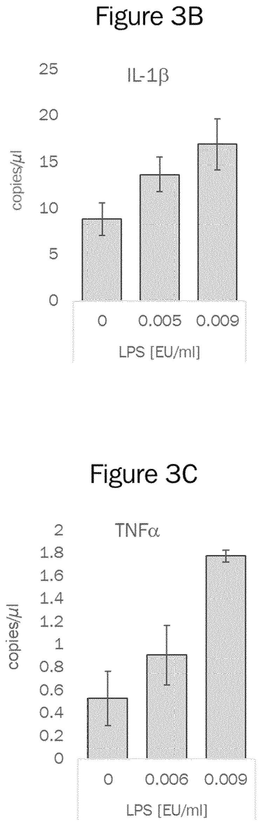

Figure 3B is a graph that represents the gene expression of IL-1β as a response in a monocyte activation assay carried out with THP-1 derived macrophages when amplified with digital PCR. A housekeeping gene TATA-binding protein (TBP) ensures data accuracy. Cytokine gene expression is to endotoxin lipopolysaccharide (LPS) from *Escherichia coli* (0, 0.005 EU/ml and 0.009 EU/ml).
Figure 3C is a graph that represents the gene expression of TNFα as a response in a monocyte activation assay carried out with THP-1 derived macrophages when amplified with digital PCR. A housekeeping gene TATA-binding protein (TBP) ensures data accuracy. Cytokine gene expression is to endotoxin lipopolysaccharide (LPS) from *Escherichia coli* (0, 0.005 EU/ml and 0.009 EU/ml).

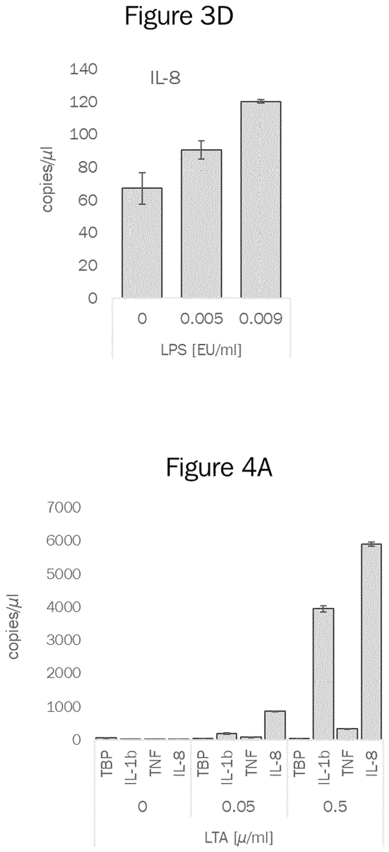

Figure 3D is a graph that represents the gene expression of IL-8 as a response in a monocyte activation assay carried out with THP-1 derived macrophages when amplified with digital PCR. A housekeeping gene TATA-binding protein (TBP) ensures data accuracy. Cytokine gene expression is to endotoxin lipopolysaccharide (LPS) from *Escherichia coli* (0, 0.005 EU/ml and 0.009EU/ml).
Figure 4A is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is to lipoteichoic acid from *Staphylococcus aureus,* a Gram-positive bacterium (0, 0.05 µg/ml and 0.05 µg/ml).

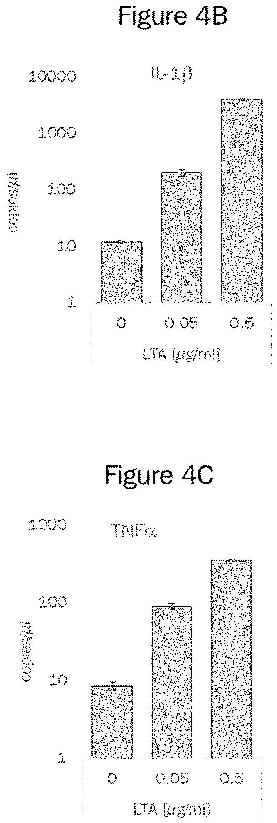

Figure 4B is a graph that represents the gene expression of IL-1β as a response in a monocyte activation assay carried out with THP-1 derived macrophages when amplified with digital PCR. A housekeeping gene TATA-binding protein (TBP) ensures data accuracy. Cytokine gene expression is to lipoteichoic acid from *Staphylococcus aureus,* a Gram-positive bacterium (0, 0.05 µg/ml and 0.05 µg/ml).
Figure 4C is a graph that represents the gene expression of TNFα as a response in a monocyte activation assay carried out with THP-1 derived macrophages when amplified with digital PCR. A housekeeping gene TATA-binding protein (TBP) ensures data accuracy. Cytokine gene expression is to lipoteichoic acid from *Staphylococcus aureus,* a Gram-positive bacterium (0, 0.05 µg/ml and 0.05 µg/ml).

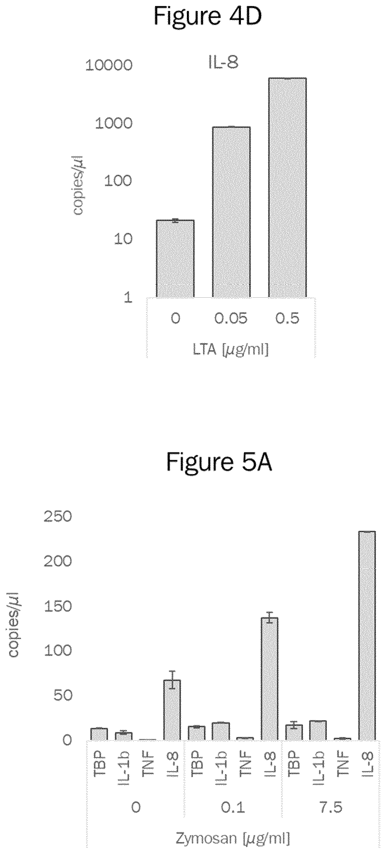

Figure 4D is a graph that represents the gene expression of IL-8 as a response in a monocyte activation assay carried out with THP-1 derived macrophages when amplified with digital PCR. A housekeeping gene TATA-binding protein (TBP) ensures data accuracy. Cytokine gene expression is to lipoteichoic acid from *Staphylococcus aureus,* a Gram-positive bacterium (0, 0.05 µg/ml and 0.05 µg/ml).
Figure 5A is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is to zymosan, a component of fungus (0, 0.1 µg/ml and 7.5 µg/ml).

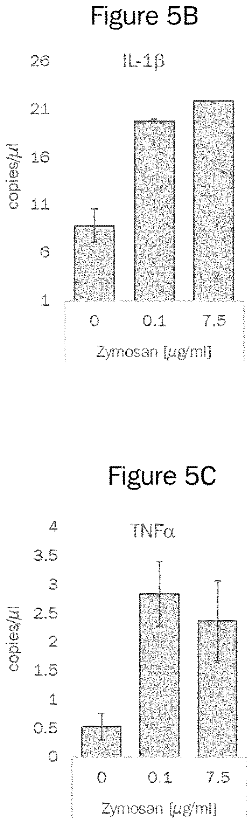

Figure 5B is a graph that represents the gene expression of IL-1β as a response in a monocyte activation assay carried out with THP-1 derived macrophages when amplified with digital PCR. A housekeeping gene TATA-binding protein (TBP) ensures data accuracy. Cytokine gene expression is to zymosan, a component of fungus (0, 0.1 µg/ml and 7.5 µg/ml).
Figure 5C is a graph that represents the gene expression of TNFα as a response in a monocyte activation assay carried out with THP-1 derived macrophages when amplified with digital PCR. A housekeeping gene TATA-binding protein (TBP) ensures data accuracy. Cytokine gene expression is to zymosan, a component of fungus (0, 0.1 µg/ml and 7.5 µg/ml).

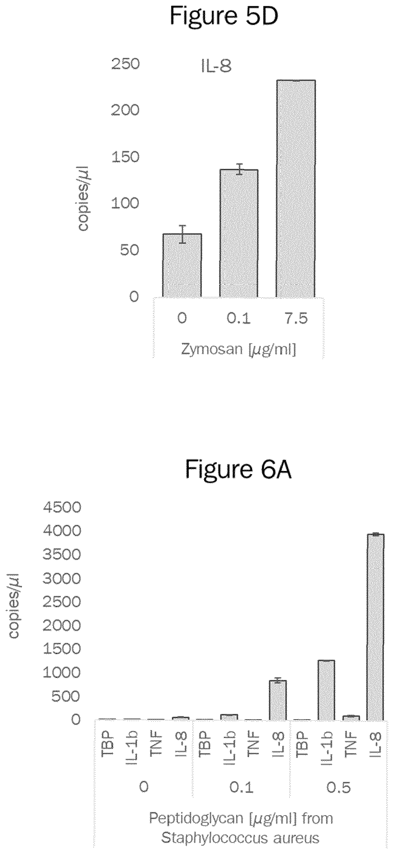

Figure 5D is a graph that represents the gene expression of IL-8 as a response in a monocyte activation assay carried out with THP-1 derived macrophages when amplified with digital PCR. A housekeeping gene TATA-binding protein (TBP) ensures data accuracy. Cytokine gene expression is to zymosan, a component of fungus (0, 0.1 µg/ml and 7.5 µg/ml).
Figure 6A is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is peptidoglycan from *Staphylococcus aureus* (0, 0.1 µg/ml and 0.5 µg/ml).

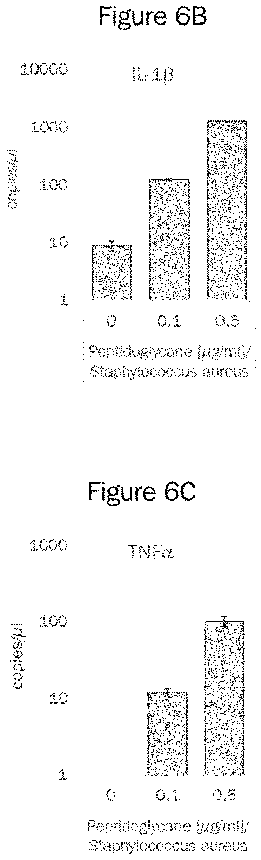

Figure 6B is a graph that represents the gene expression of IL-1β as a response in a monocyte activation assay carried out with THP-1 derived macrophages when amplified with digital PCR. A housekeeping gene TATA-binding protein (TBP) ensures data accuracy. Cytokine gene expression is peptidoglycan from *Staphylococcus aureus* (0, 0.1 µg/ml and 0.5 µg/ml).
Figure 6C is a graph that represents the gene expression of TNFα as a response in a monocyte activation assay carried out with THP-1 derived macrophages when amplified with digital PCR. A housekeeping gene TATA-binding protein (TBP) ensures data accuracy. Cytokine gene expression is peptidoglycan from *Staphylococcus aureus* (0, 0.1 µg/ml and 0.5 µg/ml).

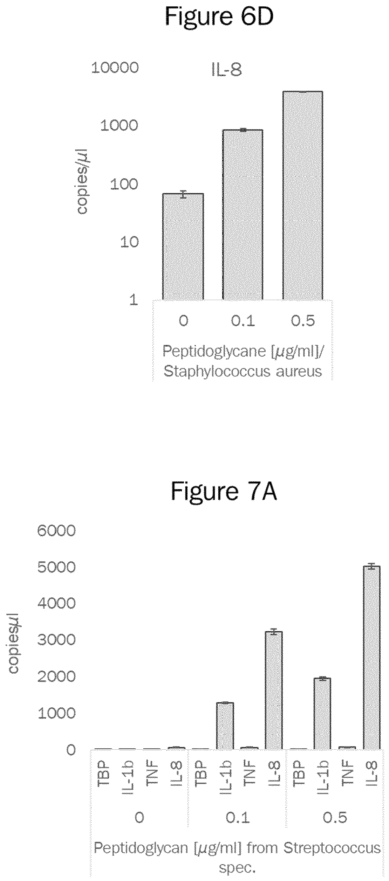

Figure 6D is a graph that represents the gene expression of IL-8 as a response in a monocyte activation assay carried out with THP-1 derived macrophages when amplified with digital PCR. A housekeeping gene TATA-binding protein (TBP) ensures data accuracy. Cytokine gene expression is peptidoglycan from *Staphylococcus aureus* (0, 0.1 µg/ml and 0.5 µg/ml).
Figure 7A is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is peptidoglycan from *Streptococcus* spec. (0, 0.1 µg/ml and 0.5 µg/ml).

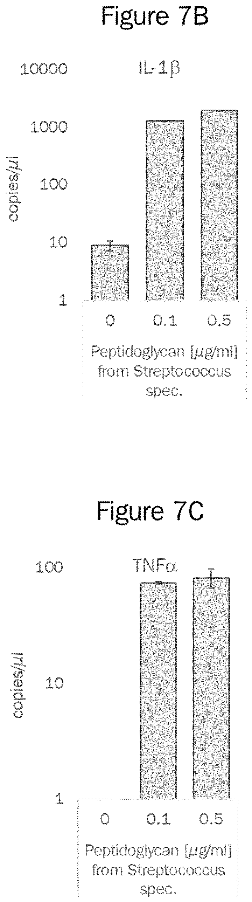

Figure 7B is a graph that represents the gene expression of IL-1β as a response in a monocyte activation assay carried out with THP-1 derived macrophages when amplified with digital PCR. A housekeeping gene TATA-binding protein (TBP) ensures data accuracy. Cytokine gene expression is peptidoglycan from *Streptococcus* spec. (0, 0.1 µg/ml and 0.5 µg/ml).
Figure 7C is a graph that represents the gene expression of TNFα as a response in a monocyte activation assay carried out with THP-1 derived macrophages when amplified with digital PCR. A housekeeping gene TATA-binding protein (TBP) ensures data accuracy. Cytokine gene expression is peptidoglycan from *Streptococcus* spec. (0, 0.1 µg/ml and 0.5 µg/ml).

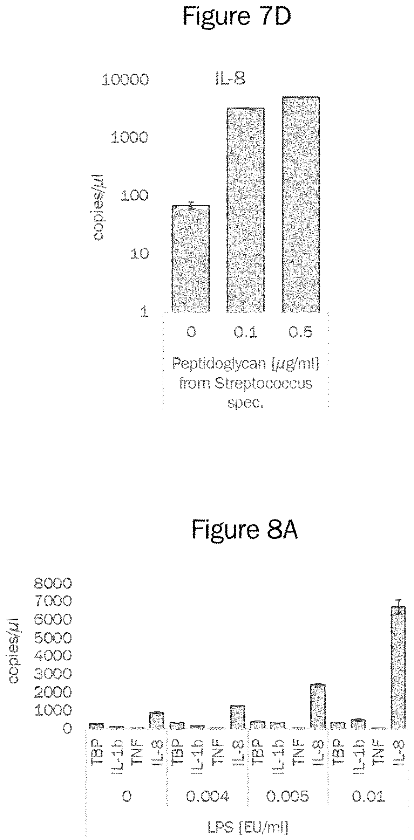

Figure 7D is a graph that represents the gene expression of IL-8 as a response in a monocyte activation assay carried out with THP-1 derived macrophages when amplified with digital PCR. A housekeeping gene TATA-binding protein (TBP) ensures data accuracy. Cytokine gene expression is peptidoglycan from *Streptococcus* spec. (0, 0.1 µg/ml and 0.5 µg/ml).
Figure 8A is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is lipopolysaccharide (0, 0.004 EU/ml, 0.005 EU/ml and 0.01 EU/ml).

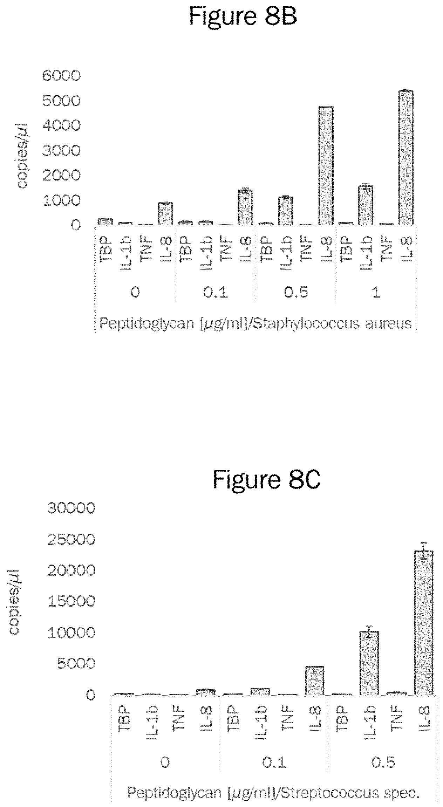

Figure 8B is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is peptidoglycan from *Staphylococcus aureus* (0, 0.1 µg/ml and 0.5 µg/ml, 1 µg/ml).
Figure 8C is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is peptidoglycan from *Streptococcus* spec. (0, 0.1 µg/ml and 0.5 µg/ml).

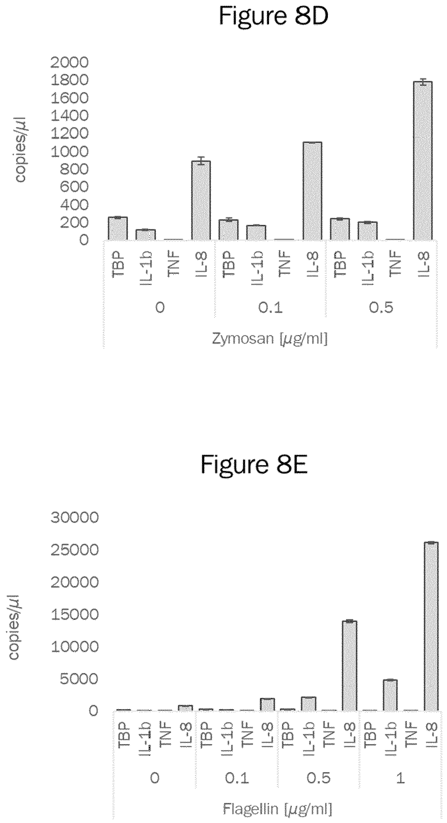

Figure 8D is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is zymosan (0, 0.1 µg/ml and 0.5 µg/ml).
Figure 8D is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is zymosan (0, 0.1 µg/ml and 0.5 µg/ml).
Figure 8E is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is flagellin (0, 0.1 µg/ml and 0.5 µg/ml, 1 µg/ml).

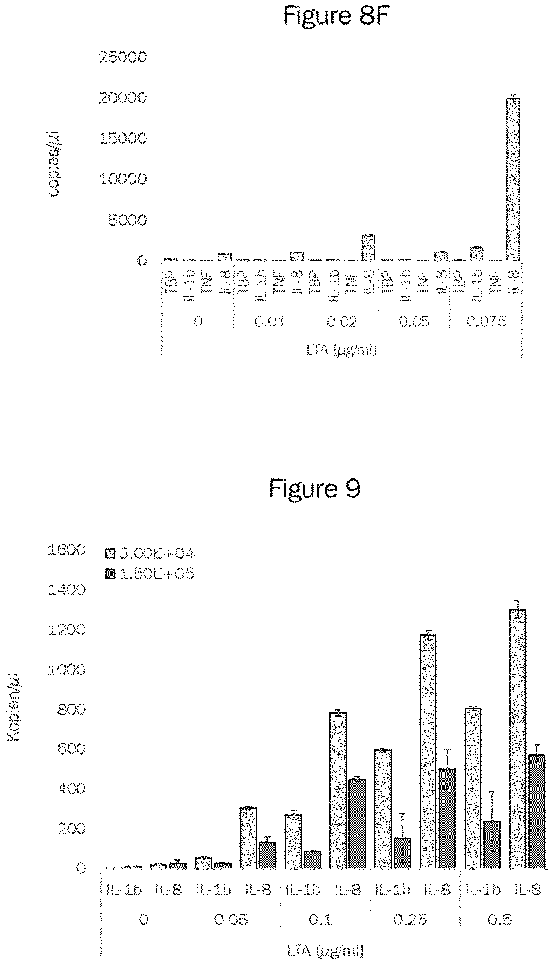

Figure 8F is a graph that represents the gene expression of IL-1β, TNFα and IL-8 as a response in a monocyte activation assay carried out with THP-1 derived macrophages when amplified with digital PCR. A housekeeping gene TATA-binding protein (TBP) ensures data accuracy. Cytokine gene expression is lipoteichoic acid (0, 0.01 µg/ml, 0.02 µg/ml, 0.05 µg and 0.075 µg/ml).
Figure 9 is a graph that represents the gene expression of IL-1þ and IL-8 as a response in a monocyte activation assay carried out with THP-1 derived macrophages with different cell densities (500,000 cells per 100 µl per 96-well, 1.5 million cells per 100 µl per 96-well) amplified with digital PCR. Cytokine gene expression is to non-endotoxin pyrogene lipoteichoic acid (LTA) in a concentration series of 0.05 µl/ml LTA, 0.1 µl/ml LTA, 0.25 µl/ml LTA and 0.5 µl/ml LTA as well as a negative control 0 µg/ml LTA).

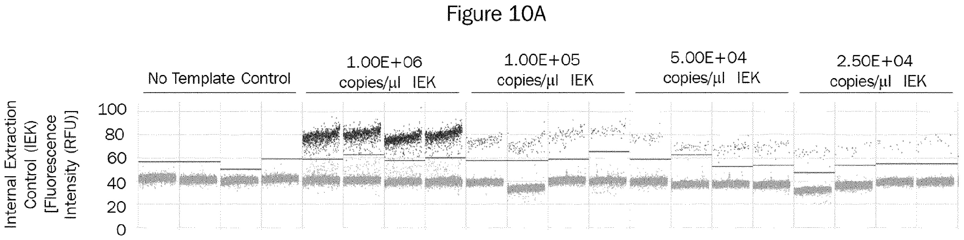

Figure 10A is a scatter plot depicting the relative fluorescence intensity from amplification with digital PCR representing the gene expression of a synthetic internal extraction control (IK_RNA) submitted to extraction in different concentrations (1 million copies per 12 µl PCR-reaction, 0.5 million copies per 12 µl PCR-reaction, 50,000 copies per 12 µl PCR-reaction and 25,000 copies per 12 µl PCR-reaction).

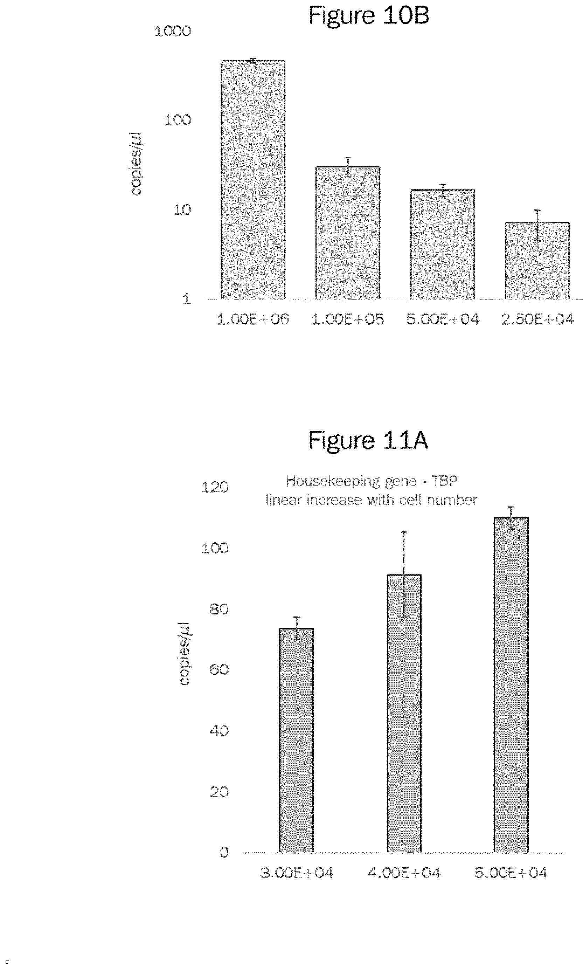

Figure 10B is a graph representing the quantification of the gene expression amplified with dPCR and calculated from the scatter plot shown in Figure 8A of a synthetic internal extraction control (IK_RNA) submitted to extraction in different concentrations (1 million copies per 12 µl PCR-reaction, 0.5 million copies per 12 µl PCR-reaction, 50,000 copies per 12 µl PCR-reaction and 25,000 copies per 12 µl PCR-reaction).
Figure 11A is a graph that depicts the base line linear increase of the gene expression of the housekeeping gene TATA Binding Protein (TBP) when different cell densities were used (30,000 cells per 100 µl per 96-well, 40,000 cells per 100 µl per 96-well, 50,000 cells per 100 µl per 96-well) amplified with digital PCR. Gene expression represents the base line in untreated control THP-1 monocyte cells.

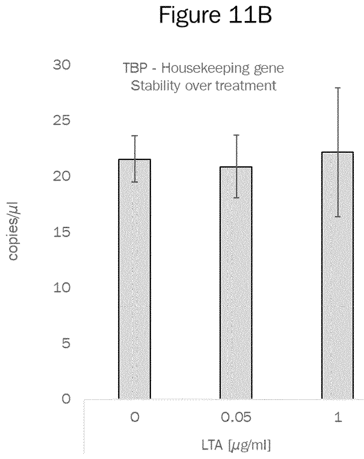

Figure 11B is a graph that depicts the gene expression of the housekeeping gene TBP amplified with digital PCR in a monocyte activation assay using THP-1 monocytes when treated with non-endotoxin LTA in three concentrations (0 µg/ml LTA, 0.05 µg/ml LTA, 1 µg/ml LTA) representing stability of TBP over the treatment.

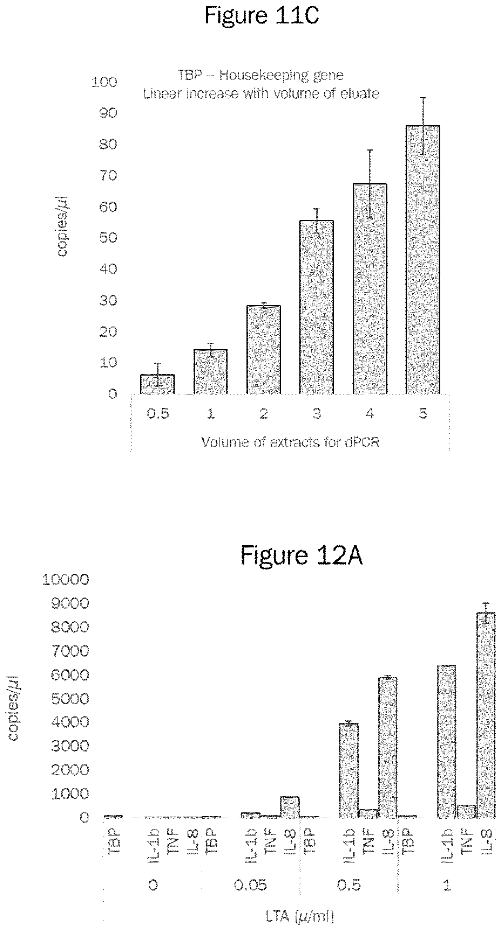

Figure 11C is a graph that represents the base line gene expression of the housekeeping gene TBP when different volumes of extracts (0.5 µl in a 12 µl dPCR-reaction, 1 µl in a 12 µl dPCR-reaction, 2 µl in a 12 µl dPCR-reaction, 3 µl in a 12 µl dPCR-reaction, 4 µl in a 12 µl dPCR-reaction, 5 µl in a 12 µl dPCR-reaction) were amplified with digital PCR showing a linear increase of gene expression with increasing volumes.
Figure 12A is a scatter plot depicting the relative fluorescence intensity from amplification with digital PCR representing the gene expression of the housekeeping gene TBP and the cytokines/chemokines IL-1β, TNFα and IL-8 as a response to non-endotoxin pyrogen LTA in THP-1 derived macrophages. Included is a no-template PCR -control (NTC).

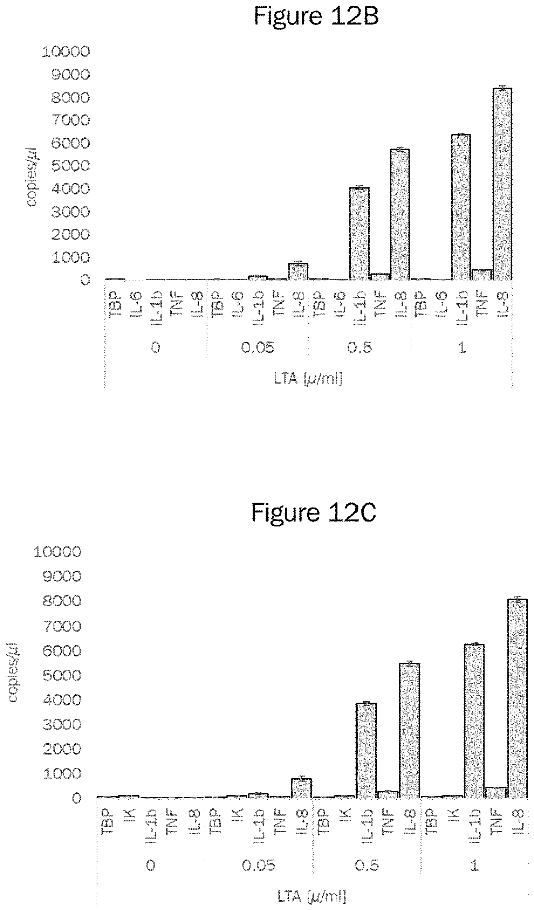

Figure 12B is a scatter plot depicting the relative fluorescence intensity from amplification with digital PCR representing the gene expression of the housekeeping gene TBP and the cytokines/chemokines IL-1β, IL-6, TNFα, IL-8 as a response to non-endotoxin pyrogen LTA in THP-1 derived macrophages. Included is a no-template PCR -control (NTC).
Figure 12C is a scatter plot depicting the relative fluorescence intensity from amplification with digital PCR representing the gene expression of the housekeeping gene TBP and the cytokines/chemokines IL-1β, TNFα and IL-8 and an internal extraction control IK_RNA as a response to non-endotoxin pyrogen LTA in THP-1 derived macrophages. Included is a no-template PCR -control (NTC).

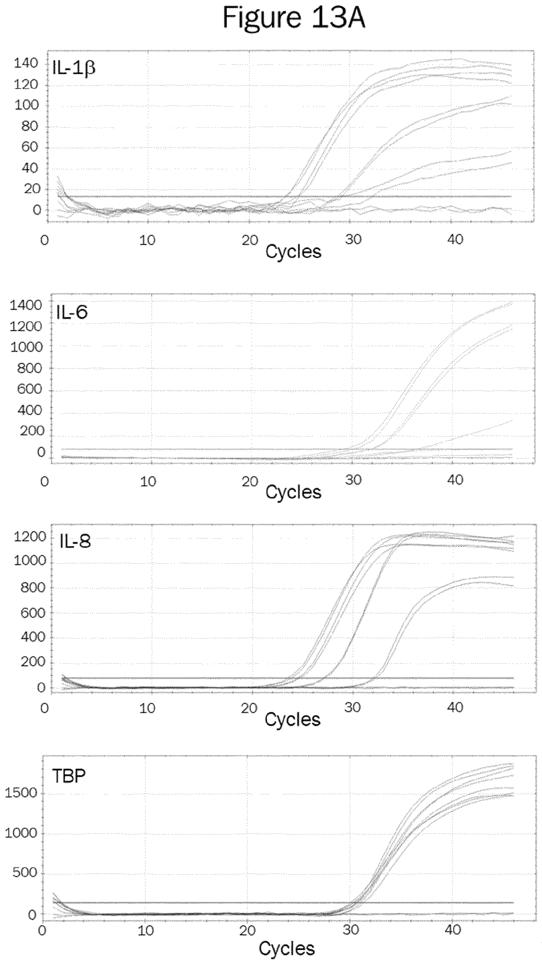

Figure 13A is a graph that depicts amplification curves of the gene expression amplified with quantitative real-time PCR (qPCR) of the cytokines/chemokines IL-1β, IL-6 and IL-8 and the housekeeping gene TBP as a response to the non-endotoxin pyrogen LTA in a monocyte amplification assay using THP-1 monocytes.

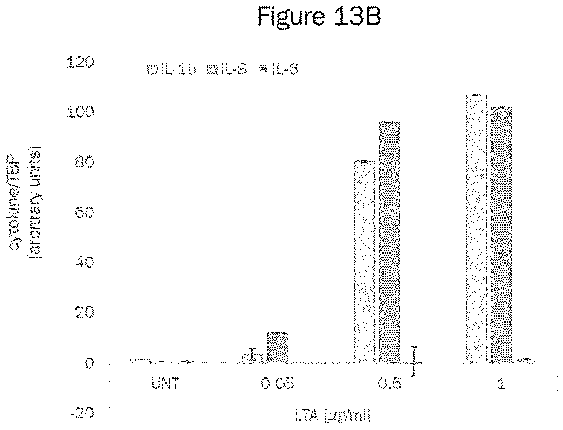

Figure 13B is graph that represents the relative gene expression of the cytokines/chemokines IL-1β, IL-6 and IL-8 over the housekeeping gene TBP correlating to the Ct-values of the curves as shown in Figure 9A.

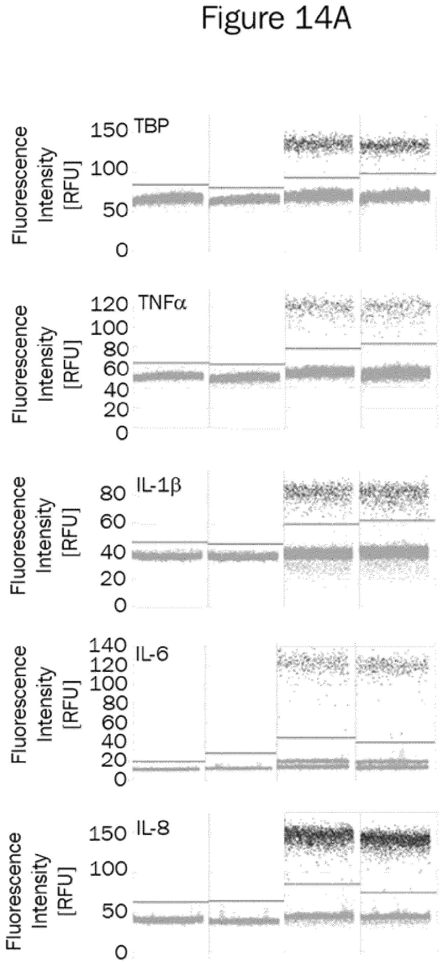

Figure 14A is a is a scatter plot depicting the relative fluorescence intensity from amplification with digital PCR representing the gene expression of five synthetic gene fragments for the cytokines/chemokines IL-1β, IL-6, TNFα and IL-8, the housekeeping gene TBP in a quintuplex functioning as a positive PCR control.

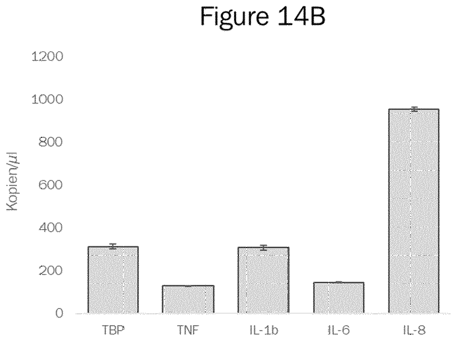

Figure 14B is a graph representing the quantification of the amplification with digital PCR representing the gene expression of five synthetic gene fragments for the cytokines/chemokines IL-1β, IL-6, TNFα and IL-8, the housekeeping gene TBP in a quintuplex functioning as a positive PCR control.

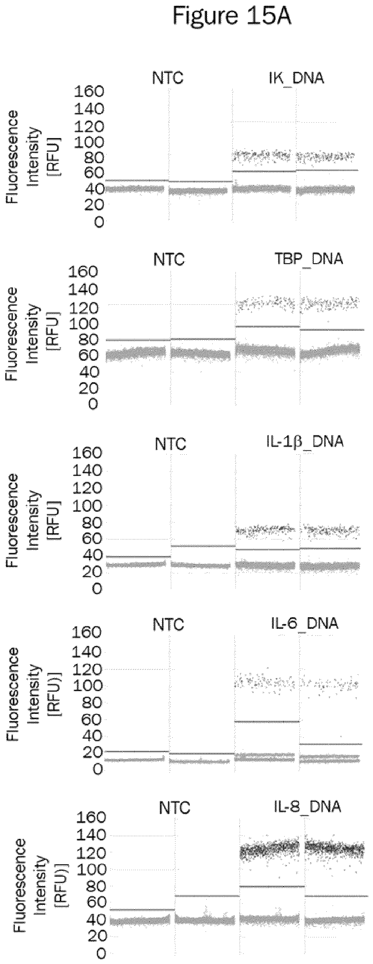

Figure 15A is a is a scatter plot depicting the relative fluorescence intensity from amplification with digital PCR representing the gene expression of five synthetic gene fragments for the cytokines/chemokines IL-1β, IL-6, and IL-8, the housekeeping gene TBP and the internal control IK_DNA equivalent to the internal extraction control IK_RNA in a quintuplex functioning as a positive PCR control.

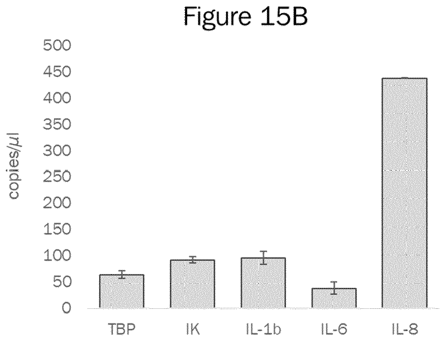

Figure 15B is a graph representing the quantification of the amplification with digital PCR representing the gene expression of five synthetic gene fragments for the cytokines/chemokines IL-1β, IL-6, and IL-8, the housekeeping gene TBP and the internal control IK_DNA equivalent to the internal extraction control IK_RNA in a quintuplex functioning as a positive PCR control.

### DETAILED DESCRIPTION OF THE INVENTION

The present invention is based on the capability of human immune cells to react to external stimuli such as pyrogens with the expression of a plethora of pro-inflammatory immuno-modulatory agents including TNFα, IL-1β, IL-6 and IL-8 among many others. The subsequent release of these expressed pro-inflammatory cytokines and chemokines cause an immuno-modulatory response of the immune system of the organism leading to an inflammatory reaction. The present invention is directed to measure the gene expression of the cytokines/chemokines TNFα, IL-1β, IL-6 and IL-8 in response to external pyrogens. For this purpose, macrophages are incubated with a medicinal sample for a few hours along with a pyrogen standard and subsequently the mRNA is isolated and the gene expression is measured by polymerase chain reaction. Two methods of polymerase chain reaction (PCR) can be applied - the quantitative real-time PCR or the digital PCR.

The here presented monocyte activation test was developed to detect endotoxin, that is Gram-negative bacteria (e. g. *Escherichia coli, Pseudomonas aeruginosa, Chlamydia trachomatis, Yersinia pestis*) and non-endotoxin pyrogens like Gram-positive bacteria (e. g. *Staphylococcus aureus, Streptococcus* spec., *Bacillus* spec., *Clostridium*) or fungus (e. g. *Saccharomyces cerevisiae*)*.* Due to the capability of monocytes/macrophages to react to a plethora of pyrogens, the invention can be utilized to detect a wide array of different biological and non-biological agents.

### 1. Pyrogen Test Components

The invention can be utilized to detect endotoxin and non-endotoxin contaminations in pharmaceutical products such as parenteral medical products, dialytes, vaccines, intravenous solutions and any other medicinal or non-medicinal fluid that are not applied orally or get in contact with bodily fluids.

### A. Endogenous immuno-modulatory mediators of the inflammatory response

In theory, any immuno-modulatory mediator that is expressed by the monocyte/macrophage system in response to pyrogens could be applied to detect pyrogen contamination including IL-1β, TNFα or IL-6 or IL-8 among others (Mäkelä et al., 2009). The cytokines IL-1β, TNFα or IL-6 are recommended as detectors in the monocyte activation assay (MAT) in the European Pharmacopoeia 07/2017:20630 chapter 2.6.30. Monocyte-Activation Test. These cytokines are known to be involved in the pathogenesis leading to fever and septic shock (Evans et al., 2015). Although the role of the chemokine IL-8 in the development of fever and sepsis is less well studied, it is produced by the monocyte/macrophage system abundantly (Duque & Descoteaux, 2014) and macrophages are the first cells in the immune response to express and secrete IL-8 to attract other immune cells such as neutrophils to the site of infection (Duque & Descoteaux, 2014). Neutrophils are the primary mediator that are involved in the rapid innate host defence to microbiological pathogens and the first defender that produce Reactive Oxygen Species (ROS) (Frøland, 1984). Since ROS can activate among others the nuclear factor-κB pathway that is an important transcription factor for cytokines and chemokines, a connection between increased ROS production and the progression of inflammatory diseases is emerging as an additional factor of the inflammatory response (Naik & Dixit, 2011). That makes IL-8 an important detector for the inflammatory response.

The present invention is based on the above-mentioned European Pharmacopoeia 07/2017:20630 chapter 2.6.30. Here, it is recommended to detect at least one of the cytokines IL-1β, TNFα or IL-6. The invention relies on the measurement of at least three cytokines/chemokines in parallel by dPCR or qPCR (IL-1β, TNFα, CXCL8/IL-8), the housekeeping gene TATA-binding protein (TBP) to control data accuracy and the additional opportunity to add either a fourth cytokine IL-6 or an internal extraction control RNA (IK_RNA) which is a synthetic RNA that can be added before the extraction of the treated monocytes/macrophages. Although immunoreactive and secreted IL-6 protein seems to be the key cytokine in the pro-inflammatory response, IL-6 mRNA in macrophages is bound by YB-1 and easily secreted to the extracellular space and thereby reducing IL-6 mRNA within the macrophage cell unlike TNFα mRNA (Kang et al., 2014). In a different immune cell type - the dendritic cells - the situation is different. Here, YB-1 functions as an IL-6 mRNA stabilator. Thus, the invention provides the detector for IL-6 gene expression optionally depending on the cell type, which is used for detection of pyrogen contaminations in medicinal products.

### B. Primer/Probe System for the detection of the endogenous immuno-modulatory mediators of the inflammatory response

After determining the endogenous immuno-modulatory mediators of the inflammatory response, the primer/probe system must be made for utilization in the present invention. Primer/probe systems are developed for detecting the gene expression that is detecting the mRNA levels of the cytokines IL-1β, IL-6, TNFα, the chemokine CXCL-8/IL-8, the housekeeping gene TBP and the synthetic mRNA IK_RNA. The primer/probe systems are specific for the respective mRNAs and do not detect genomic DNA with which the samples could be contaminate. The primer/probe systems are supposed to be simultaneously amplified by either dPCR or qPCR in one reaction.

Four primer/probe systems are obligatory components of a master mix containing an assay reagent and a reverse transcriptase provided by QIAGEN to be used in the QIAcuity-dPCR system (QIAGEN). A fifth optional primer/probe set can be added, either the primer/probe system for the detection of IL-6 or the primer/probe system for the detection of the synthetic RNA (IK_RNA). The master mix can be equally used in any qPCR system that allows the detection of at least four mRNAs simultaneously (e. g. CFX96^{™} Real-Time System by BioRad).

### C. Monocyte/Macrophage Cell System

The first line in the Nucleic Acid Amplification Test Monocyte Activation Assay (NAT-MAT) is the immuno-competent cell system. The present invention relies on the monocyte/macrophage system, which are combined with the sample to be tested. Basically, all cells that can express Toll-like receptors (TLRs) or were made to express TLRs can be used for the application of the invention. TLRs are the mediators of the inflammatory response to the pyrogens. They are preferably of the same origin as for whom the medicinal products are intended to be used, that means human monocytes/macrophages for pharmaceutical products, cat, dog, mouse etc. cells for veterinary products. Cells are seeded in wells of a 96-well cell culture plate.

One embodiment of the invention is the monocytic cell line THP-1. They need to be present in the cell density of at least 30 000 cells per 96-well, but also works at cell densities of at least 40 000 cells per 96-well, 50 000 cells per 96-well, 60 000 cells per 96-well, 70 000 cells per well, 80 000 cells per well, 90 000 cells per well or 100 000 cells per well. Other monocytic cell lines could be equally used.

Another embodiment of the invention are THP-1 derived macrophages. THP-1 derived macrophages are more competent to react to pyrogens than monocytes. In a first step, monocytes are triggered to differentiate to macrophages. Macrophages are provided with the invention and seeded in a 96-well cell culture plate. They need to be present in the cell density of at least 30 000 cells per 96-well, but also works at cell densities of at least 40 000 cells per 96-well, 50 000 cells per 96-well, 60 000 cells per 96-well, 70 000 cells per well, 80 000 cells per well, 90 000 cells per well or 100 000 cells per well. THP-1 derived macrophages need to be given at least 24 hours to attach to the surface of the 96-well cell culture plate.

### 2. The Assay System

The pyrogenicity assay is carried out in three different containers.

### A. Seeding and treatment of the monocytes/macrophages with the samples and a pyrogen standard

In a first container that is a 96-well cell culture plate, THP-1 monocytes or THP-1 derived macrophages are seeded and incubated with the sample and a pyrogen standard concentration series for different length of time. The volumes in which the samples are added are at least 50 µl and not more than 150 µl. Ideally the incubation time ranges from two to four hours. Incubation of the cells with the sample is carried out in the same container as where the cells were seeded.

### B. Extraction for obtaining the mRNAs for the detection of the endogenous immuno-modulatory mediators of the inflammatory response

After the incubation time is completed, three times the volume of a lysis buffer are added to the 96-well cell culture plate. The assay container is subsequently subjected to an automated extraction system (e. g. KingFisher Flex from Thermo Fisher Scientific) and either a mixture of total DNA and total RNA is obtained as an eluate or a mixture of total RNA as an eluate depending on the extraction system that is used (e. g. modified AniPath-Kit from IST Innuscreen).

### C. Quantitative Polymerase Chain Reaction for the detection of the endogenous immuno-modulatory mediators of the inflammatory response

Both, digital PCR and real-time PCR are methods where the amounts of nucleic acids can be determined quantitatively. Both methods are sensitive and precise. They distinguish themselves by the read-out. The newly emerged digital PCR is similar to traditional PCR an endpoint PCR unlike quantitative real-time PCR where the reaction progress is monitored. Digital PCR does not require a standard curve for the quantitative determination of nucleic acids. For the quantification of the nucleic acids amplified by real time PCR, a standard curve or a reference, i.e., a housekeeping gene is required, enabling relative quantification. Digital PCR provides absolute quantitative determination of nucleic acids and does not require a standard curve.

The eluates are subjected to dPCR or qPCR in a reaction mix comprising a PCR puffer, a reverse transcriptase for reverse transcribing mRNA and a primer/probe system to detect IL-1β, IL-8, TNFα and a housekeeping gene TBP simultaneously with the option to add an additional primer/probe system to detect either IL-6 or a synthetic RNA (IK_RNA) that would be added to the cells before extraction.

According to the requirements of the European Pharmacopoeia 07/2017:20630 chapter 2.6.30. Monocyte-Activation Test a pyrogen standard in a concentration series is added to the cell system along with the sample and amplified.

### 3. Detection Kit

The above-described embodiments can be incorporated into a diagnostic kit for the detection of pyrogen contaminations in medicinal products or to perform a pyrogen test. The kit can detect endotoxin pyrogens from Gram-negative bacteria and non-endotoxin pyrogens from Gram-positive bacteria and their components (e. g., lipoteichoic acid, peptidoglycan, flagellin) and fungus or their components (e. g., zymosan).

The kit can be employed to detect endotoxin and non-endotoxin contaminations in pharmaceutical products such as parenteral medical products, dialytes, vaccines, intravenous solutions and any other medicinal or non-medicinal fluid that are not applied orally or get in contact with bodily fluids.

### 4. Interpretation of Cytokine/Chemokine Gene Expression Data

The interpretation and the analysis of the gene expression data obtained by dPCR are analysed according to the requirements of the European Pharmacopoeia 07/2017:20630 chapter 2.6.30. Monocyte-Activation Test.

### A. Quantitative Test using the Standard Curve Method

The quantitative test utilizes a pyrogen standard curve in concentrations recommended by the European Pharmacopoeia 07/2017:20630 chapter 2.6.30. Monocyte-Activation Test. This standard curve is generated by incubating the monocytes/macrophages with the endotoxin USP reference standard. This enables the quantification of the produced cytokines in response to the activation of the monocytes/macrophages in comparison to a known endotoxin standard. Any other pyrogen that is available can be used for generating the standard curve. The standard curve can be generated with any data points that are generated with significantly varied concentrations of standard endotoxin or any other known pyrogen by employing standard best-fit data analysis software. The European Pharmacopoeia 07/2017:20630 chapter 2.6.30. Monocyte-Activation Test suggests for generation of the standard curve five data points ranging from R0 (no treatment; negative control), and R1 to R4. The R1 data point represents the 0.5-x limit of detection for the known pyrogen used in the standard curve, R2 represents the 1-x limit of detection for the known pyrogen used in the standard curve, R3 represents the 2-x limit of detection for the known pyrogen used in the standard curve and R4 represents the 4-x limit of detection for the known pyrogen used in the standard curve. After generating the standard curve, the equivalent endotoxin or non-endotoxin concentrations of the sample to be tested can be interpolated from the standard curve. By utilizing the USP reference standard helps to normalize the obtained data to endotoxin units (EU) per milliliter) as defined by the USP/FDA which are identical to international units (IU) as was defined by the World Health Organization and are the industry standard units for indicating pyrogen contamination.

### DEFINITIONS

The term endotoxin as used herein refers to the endotoxin lipopolysaccharide (LPS).

The term non-endotoxin pyrogen as used herein refers to all other pyrogens that are not the endotoxin lipopolysaccharide (LPS).

The term assay system as used herein refers to any cell culture plate containing monocytes/macrophages including the sample to be tested, the standard and the primer/probe system that is used for detection of the cytokine gene expression, the gene expression the house keeping gene, and the internal extraction control IK_RNA.

### MATERIALS AND METHODS

### Cell culture of THP-1 monocytes.

THP-1 monocytes (CLS Cell Lines Service GmbH) are cultured in RPMI1640 medium with glutamine and sodium bicarbonate (Capricorn Scientific) supplemented with sodium pyruvate (Sigma-Aldrich), HEPES (Gibco) and glucose to obtain a concentration of 3.5 g/Lglucose (Capricorn Scientific), heat-inactivated fetal bovine serum (Sigma-Aldrich) and β-mercaptoethanol (PAN Biotech) in a cell density of 10,000 to 50,000 cells per milliliter in an enviroment of 37 °C and 5% CO₂.

### Preparation of THP-1 derived macrophages.

THP-1 monocytes are cultivated in RPMI1640 medium with glutamine and sodium bicarbonate (Sigma-Aldrich) supplemented with sodium pyruvate (Sigma-Aldrich), HEPES (Gibco) and glucose (Capricorn Scientific), heat-inactivated fetal bovine serum (Sigma-Aldrich) and β-mercaptoethanol (PAN Biotech). 15,000,000 of the THP-1 monocytic suspension cells were counted and treated with 10 to 20 nM Phorbol_12_myristate_13_acetate (PMA) (Sigma-Aldrich) for four to eight days for differentiation of the THP-1 monocytes to THP-1 derived macrophages in T175 cell culture flask (area 175 cm²) in a 37 °C incubator with 5% CO₂.

### Cell seeding of THP-1 derived macrophages.

THP-1 derived macrophages were seeded in quadruplicates of a 96-well plate with each well of the quadruplicate containing 50,000 cells (cell density ranging from 50,000 to 150,000 cells per well) in RPMI1640 medium with glutamine and sodium bicarbonate (Sigma-Aldrich) supplemented with sodium pyruvate (Sigma-Aldrich), HEPES (Gibco) and glucose (Capricorn Scientific), heat-inactivated fetal bovine serum (Sigma-Aldrich) and β-mercaptoethanol (PAN Biotech). Treatment of the cells with the standard will be performed in the same matrix in which the sample to be tested is dissolved, usually physiologic sodium chloride solution in a volume of 100 µl for one to four hours. The sample was diluted according to the requirements of the European Pharmacopoeia 07/2017:20630 chapter 2.6.30. Monocyte-Activation Test. The sample to be tested will be applied to the cells undiluted, 2 x dilution and 4 x dilution as well as the same dilutions spiked with the middle dose of the standard.

### Extraction of the mRNA from THP-1 derived macrophages after treatment with the standard pyrogen and the sample to be tested.

After the incubation time, the mRNA is extracted to measure the gene expression of the cytokines and the housekeeping gene. For this purpose, a lysis buffer containing guanidinthiocyanate is added in equal volume (100 µl) to the cells in the cell culture plate. This causes the detachment of the cells from the cell culture plate surface. Lysed cells are transferred to a deep well plate (KFFLX Plate Set, #845-KF-1296010, IST Innuscreen) for automatic extraction in the KingFisher Flex system (Thermo Fisher Scientific). Nucleic acids (DNA and RNA) are captured by magnetic beads (#31-00641 IST Innuscreen), washed and eluted in RNAse-free water (#314-00851, IST Innuscreen) into an elution plate.

### Digital polymerase chain reaction (dPCR).

The extracts were subjected to amplification with the digital PCR (QIAcuity, QIAGEN). For this, we used a one-step dPCR kit combining reverse transcription of the messenger RNA (mRNA) to complementary DNA (cDNA). A reaction mix containing a probe master mix, a reverse transcriptase, gene specific primer/probe mix containing specific primers and probes for the cytokines/chemokines IL-1β, TNFα, IL-8 and TBP and 6 µl of the extracts containing RNA to a total volume of 12 µl is transferred to one well an 8.5k 96-well nanoplate (#250011, QIAGEN) for amplification. The reaction mix is pipetted directly from the elution plate containing the extracts to the nanoplate by the aid of the pipetting robot QIAgility (QIAGEN). The amplification was performed with the aid of digital PCR in the thermocycler QIAcuity (QIAGEN). Results are given as copies per microliter calculated by the integrated analysis software QIAcuity Suite.

### Interpretation of cytokine production data.

Medicinal products are tested in different dilutions and the results will be quantitatively analysed according to the requirements of the European Pharmacopoeia 07/2017:20630 chapter 2.6.30. Monocyte-Activation Test.

### A. Standard curve method - qualitative method.

First of all, the validation of the standard curve consisting of R0 to R4 has to be confirmed according to the following requirements: i) The regression of responses on log10 dose shall be statistically significant (p<0.01). ii) The regression of responses on log10 dose must not deviate significantly from linearity (p>0.05). In a first step, the suitable dilution of the sample, which was added undiluted and in a twofold and fourfold dilution, for the analysis has to be determined. Two different samples can be tested simultaneously on one 96-well cell culture plate in one assay; each with three dilutions (undiluted, twofold and fourfold diluted) and for each sample the equivalents in each of the replicates has to suit following criteria: i) Average of the four replicates has to be calculated and the recovery of the endotoxin calculated - that results from the subtraction of the mean concentration without spike from the mean concentration of the sample dilution with added spike R3. This has to be in the range of 50 - 200 %. ii) The dilutions that do not meet the spike recovery criteria will not be considered in the further analysis; if none of the dilutions meet these criteria, the assay cannot be analysed and different dilutions have to be reconsidered. iii) The sample that will be examined has to comply with the requirements of endotoxin equivalents measured in the samples without spike after former correction for dilution and concentration have to be less than the contaminant limit concentration (CLC) specified for the sample being examined in order to be considered negative. iv) Further negative controls that are added to the PCR-reaction in order to check the purity of the used reagents need a result of 0 copies per microliter for the assay to be valid for analysis.

### NAT-MAT Sequences

| | |
|---|---|
| IK_FW | CACGCTATCGCTGCCTCTAT |
| IK_RV_2 | CGGCTATTGAGATACACTGATC |
| dsIK_P211 HEX | HEX-AGCTATCACGCTCATGCG-BHQ1 |
| IL-1b_FW_kurz | ATCTTTGAAGAAGAACCTATC |
| IL-1b_kurz_RV_2 | CTGTGAGTCCCGGAGC |
| IL-1b_TAMRA | TAMRA-TAACGAGGCTTATGTGCACGATG-BHQ2 |
| IL-8_exov_FW_2 | CTTTCAGAGACAGCAGAGCAC |
| IL-8_exov_RV | CTTGGCAAAACTGCACCTTCAC |
| IL-8_Cy5 | Cy5-CGTGGCTCTCTTGGCAGC-BHQ2 |
| IL-6_FW_kurz | CATGTAACAAGAGTAACATGTGT |
| IL-6_exov_RV | CACCAGGCAAGTCTCCTCAT |
| IL-6_ROX_S2 | ROX-AGCCATCTTTGGAAGGTTCAGG_BHQ2 |
| TBP_FW | CTTTGCAGTGACCCAGCATC |
| TBP_RV | TCTTGGCAAACCAGAAACCCT |
| TBP_FAM | FAM-CTGTTTCTTGGCGTGTGAAGATAACCC-BHQ1 |
| TNF FW 2 | TGCTTGTTCCTCAGCCTCT |
| TNF RV 2 | CTTGAGGGTTTGCTACAACA |
| TNFalpha PR2 | ROX-ACGCTCTTCTGCCTGCTGCA-BHQ2 |

The method according to the invention comprises the following embodiments:
1. A method of detecting endotoxin or non-endotoxin pyrogens in a sample comprising following steps:
   the seeding of cryopreserved monocytes/macrophages in 96-well cell culture plates at an appropriate cell density
   the combination of the sample with the monocyte/macrophage system that is capable of responding to the sample with the production of certain pro-inflammatory cytokines
   the addition of a lysis buffer to the cells after an incubation time of two to four hours and
   transferring the monocyte/macrophage containing cell culture plate to an automated extraction system that utilises magnetic beads to which nucleic acids can bind and be eluted after being washed and purified from cell debris
   the transferral of the eluates containing nucleic acids DNA/RNA to an automated pipetting system to combine PCR reagents comprising apart from an enzyme that is capable of amplifying DNA, a buffer in which the enzyme can work, the primer/probe system containing primer/probes for three cytokines/chemokines IL-1β, TNFα, IL-8 and the primer/probe for a housekeeping gene TBP, and the eluates and the transferral to an 8.5k 96-well nanoplate
   the transferral of the nanoplate to the dPCR thermocycler and the subsequent analysis of the gene expression of certain pro-inflammatory cytokines where an elevated level of at least one of the three fever-relevant cytokines/chemokines (IL-1β, TNFα, IL-8) indicates the presence of a pyrogen in the sample.
2. A method of detecting endotoxin or non-endotoxin pyrogens in a sample comprising following steps:
   the seeding of cryopreserved monocytes/macrophages in 96-well cell culture plates at an appropriate cell density
   the combination of the sample with the monocyte/macrophage system that is capable of responding to the sample with the production of certain pro-inflammatory cytokines/chemokines
   the addition of a lysis buffer to the cells after an incubation time of two to three hours and transferring the monocyte/macrophage containing cell culture plate to an automated extraction system that utilises magnetic beads to which nucleic acids can bind and be eluted after being washed and purified from cell debris
   the transferral of the eluates containing nucleic acids DNA/RNA to an automated pipetting system to combine PCR reagents comprising apart from an enzyme that is capable of amplifying DNA, a buffer in which the enzyme can work, the primer/probe system containing primer/probes for four cytokines/chemokines IL-1β, TNFα, IL-8, IL-6 and the primer/probe for a housekeeping gene TBP, and the eluates and the transferral to an 8.5k 96-well nanoplate
   the transferral of the nanoplate to the dPCR thermocycler and the subsequent analysis of the gene expression of certain pro-inflammatory cytokines/chemokines where an elevated level of at least one of the four fever-relevant cytokines/chemokines (IL-1β, TNFα, IL-8, IL-6) indicates the presence of a pyrogen in the sample.
3. A method of detecting endotoxin or non-endotoxin pyrogens in a sample comprising following steps:
   the seeding of cryopreserved monocytes/macrophages in 96-well cell culture plates at an appropriate cell density
   the combination of the sample with the monocyte/macrophage system that is capable of responding to the sample with the production of certain pro-inflammatory cytokines/chemokines
   the addition of an internal extraction control that is a synthetic RNA molecule to each well of the 96-well plate containing monocytes/macrophages
   the addition of a lysis buffer to the cells after an incubation time of two to three hours and
   transferring the monocyte/macrophage containing cell culture plate to an automated extraction system
   the transferral of the eluates to an automated pipetting system to combine PCR reagents comprising apart from an enzyme that is capable of amplifying DNA, a buffer in which the enzyme can work, the primer/probe system containing primer/probes for four cytokines/chemokines IL-1β, TNFα, IL-8, IL-6 and the primer/probe for a housekeeping gene TBP, and the eluates and the transferral to an 8.5k 96-well nanoplate
   the transferral of the nanoplate to the dPCR thermocycler and the subsequent analysis of the gene expression of certain pro-inflammatory cytokines/chemokines where an elevated level of at least one of three fever-relevant cytokines/chemokines (IL-1β, TNFα, IL-8) indicates the presence of a pyrogen in the sample
4. A method of detecting endotoxin or non-endotoxin pyrogens in a parenteral-applied medicinal product comprising following steps:
   the seeding of cryopreserved monocytes/macrophages in 96-well cell culture plates at an appropriate cell density
   the combination of the medicinal product with the monocyte/macrophage system that is capable of responding to the sample with the production of certain pro-inflammatory cytokines/chemokines
   the addition of a lysis buffer to the cells after an incubation time of two to three hours and transferring the monocyte/macrophage containing cell culture plate to an automated extraction system that utilises magnetic beads to which nucleic acids can bind and be eluted after being washed and purified from cell debris
   the transferral of the eluates containing nucleic acids DNA/RNA to an automated pipetting system to combine PCR reagents comprising apart from an enzyme that is capable of amplifying DNA, a buffer in which the enzyme can work, the primer/probe system containing primer/probes for three cytokines/chemokines IL-1β, TNFα, IL-8 and the primer/probe for a housekeeping gene TBP, and the eluates and the transferral to an 8.5k 96-well nanoplate
   the transferral of the nanoplate to the dPCR thermocycler and the subsequent analysis of the gene expression of certain pro-inflammatory cytokines/chemokines where an elevated level of at least one of the three fever-relevant cytokines/chemokines (IL-1β, TNFα, IL-8) indicates the presence of a pyrogen in the sample.
5. A method of detecting endotoxin or non-endotoxin pyrogens in a parenteral-applied medicinal product comprising following steps:
   the seeding of cryopreserved monocytes/macrophages in 96-well cell culture plates at an appropriate cell density
   the combination of the sample with the monocyte/macrophage system that is capable of responding to the sample with the production of certain pro-inflammatory cytokines/chemokines
   the addition of a lysis buffer to the cells after an incubation time of two to three hours and transferring the monocyte/macrophage containing cell culture plate to an automated extraction system that utilises magnetic beads to which nucleic acids can bind and be eluted after being washed and purified from cell debris
   the transferral of the eluates containing nucleic acids DNA/RNA to an automated pipetting system to combine PCR reagents comprising apart from an enzyme that is capable of amplifying DNA, a buffer in which the enzyme can work, the primer/probe system containing primer/probes for four cytokines/chemokines IL-1β, TNFα, IL-8, IL-6 and the primer/probe for a housekeeping gene TBP, and the eluates and the transferral to an 8.5k 96-well nanoplate
   the transferral of the nanoplate to the dPCR thermocycler and the subsequent analysis of the gene expression of certain pro-inflammatory cytokines/chemokines where an elevated level of at least one of the four fever-relevant cytokines/chemokines (IL-1β, TNFα, IL-8, IL-6) indicates the presence of a pyrogen in the sample.
6. A method of detecting endotoxin or non-endotoxin pyrogens in a parenteral-applied medicinal product comprising following steps:
   the seeding of cryopreserved monocytes/macrophages in 96-well cell culture plates at an appropriate cell density
   the combination of the sample with the monocyte/macrophage system that is capable of responding to the sample with the production of certain pro-inflammatory cytokines/chemokines
   the addition of an internal extraction control that is a synthetic RNA molecule to each well of the 96-well plate containing monocytes/macrophages
   the addition of a lysis buffer to the cells after an incubation time of two to three hours and transferring the monocyte/macrophage containing cell culture plate to an automated extraction system
   the transferral of the eluates to an automated pipetting system to combine PCR reagents comprising apart from an enzyme that is capable of amplifying DNA, a buffer in which the enzyme can work, the primer/probe system containing primer/probes for four cytokines/chemokines IL-1β, TNFα, IL-8, IL-6 and the primer/probe for a housekeeping gene TBP, and the eluates and the transferral to an 8.5k 96-well nanoplate
   the transferral of the nanoplate to the dPCR thermocycler and the subsequent analysis of the gene expression of certain pro-inflammatory cytokines/chemokines where an elevated level of at least one of three fever-relevant cytokines/chemokines (IL-1β, TNFα, IL-8) indicates the presence of a pyrogen in the sample

The *in vitro* pyrogen test works as follows:
Example 1:
   (a) Combining a monocyte/macrophage system and a sample to be tested in a cell culture plate
   (b) Incubating the monocyte/macrophage system and the sample, so that, when the sample contains a pyrogen, the monocyte/macrophage system responds by expressing a pro-inflammatory cytokine
   (c) Transferring the contents of the combined monocyte/macrophage system and the sample to an automated extraction system containing magnetic beads to which nucleic acids bind which are eluted in a later step
   (d) Transferring the eluate to a 8.5k 96-well nanoplate along with a PCR-reagent containing all reagents needed to amplify the genes of interest including primer/probe systems that enable the amplification of at least one of the fever-related pro-inflammatory cytokines/chemokines IL-1β, TNFα, IL-8 and a housekeeping gene TBP
   (e) Assaying the amplificates for the presence of cytokines/chemokines whereby an elevation of one of the tested cytokines/chemokines over baseline indicates the presence of a pyrogen.
Example 2:
   (a) Combining a monocyte/macrophage system and a sample to be tested in a cell culture plate
   (b) Incubating the monocyte/macrophage system and the sample, so that, when the sample contains a pyrogen, the monocyte/macrophage system responds by expressing a pro-inflammatory cytokine/chemokine
   (c) Transferring the contents of the combined monocyte/macrophage system and the sample to an automated extraction system containing magnetic beads to which nucleic acids bind which are eluted in a later step
   (d) Transferring the eluate to a 8.5k 96-well nanoplate along with a PCR-reagent containing all reagents needed to amplify the genes of interest including primer/probe systems that enable the amplification of at least one of the fever-related pro-inflammatory cytokines IL-1β, TNFα, IL-8, IL-6 and a housekeeping gene TBP
   (e) Assaying the amplificates for the presence of cytokines whereby an elevation of one of the tested cytokines indicate the presence of a pyrogen.
Example 3:
   (a) Combining a monocyte/macrophage system and a sample to be tested in a cell culture plate
   (b) Incubating the monocyte/macrophage system and the sample, so that, when the sample contains a pyrogen, the monocyte/macrophage system responds by expressing a pro-inflammatory cytokine
   (c) Transferring the contents of the combined monocyte/macrophage system and the sample to an automated extraction system containing magnetic beads to which nucleic acids bind which are eluted in a later step
   (d) Transferring the eluate to a 8.5k 96-well nanoplate along with a PCR-reagent containing all reagents needed to amplify the genes of interest including primer/probe systems that enable the amplification of at least one of the fever-related pro-inflammatory cytokines IL-1β, TNFα, IL-8, of an internal extraction control and a housekeeping gene TBP
   (e) Assaying the amplificates for the presence of cytokines whereby an elevation of one of the tested cytokines indicate the presence of a pyrogen.

The sample in examples 1 to 3 is a pharmaceutical, a nutrient or a medicinal product.

The *in vitro* pyrogen test can detect two pro-inflammatory cytokines IL-1β, TNFα and a chemokine CXCL8/IL-8.

The *in vitro* pyrogen test can also be based on three pro-inflammatory cytokines IL-1β, TNFα, IL-6 and a chemokine CXCL8/IL-8.

It is also possible to detect three proinflammatory cytokines IL-1β, TNFα, IL-8.

The *in vitro* pyrogen test is quality controlled by a housekeeping gene TBP or by an internal extraction control.

In one embodiment of the *in vitro* pyrogen test, the monocyte/macrophage system consists of human THP-1 cells, a monocytic cell line isolated from the peripheral blood of a patient with acute monocytic leukaemia.

In the in vitro pyrogen test, the monocyte/macrophage system comprises a sufficient number of cells to provide at least 50,000 cells per well of the test system. The detection kit for use in the *in vitro* pyrogen test includes, among other things, a microtitre plate, a dPCR-nanoplate, a cryopreserved monocyte/macrophage system, a PCR reagent mix and a standard sample.

### Literature

A, P., ER, W., & G, W.-F. (1999). [Further development of a cell culture model for the detection of bacterial pyrogens]. ALTEX, 16(1), 3-8. https://pubmed.ncbi.nlm.nih.gov/11148756/
Auwerx, J. (1991). The human leukemia cell line, THP-1: A multifacetted model for the study of monocyte-macrophage differentiation. Experientia, 47(1), 22-31. https://doi.org/10.1007/BF02041244
Duque, G. A., & Descoteaux, A. (2014). Macrophage cytokines: involvement in immunity and infectious diseases. Frontiers in Immunology, 5, 1-12.
Eperon, S., de Groote, D., Werner-Felmayer, G., & Jungi, T. W. (1997). Human monocytoid cell lines as indicators of endotoxin: Comparison with rabbit pyrogen and Limulus amoebocyte lysate assay. Journal of Immunological Methods, 207(2), 135-145. https://doi.org/10.1016/S0022-1759(97)00112-9
Eperon, S., & Jungi, T. W. (1996). The use of human monocytoid lines as indicators of endotoxin. Journal of Immunological Methods, 194(2), 121-129. https://doi.org/10.1016/0022-1759(96)00073-7
Evans, S. S., Repasky, E. A., & Fisher, D. T. (2015). Fever and the thermal regulation of immunity: the immune system feels the heat. Nature Reviews Immunology, 15, 335-349.
Fennrich, S., Fischer, M., Hartung, T., Lexa, P., Montag-Lessing, T., Sonntag, H. G., Weigandt, M., & Wendel, A. (1999). Detection of endotoxins and other pyrogens using human whole blood. Developments in Biological Standardization, 101, 131-139.
Frøland, S. S. (1984). Bacterial infections in the compromised host. Scandinavian Journal of Infectious Diseases Supplement, 43, 7-16.
Hartung, T., Aaberge, I., Berthold, S., Carlin Gunnar, Charton, E., Coecke, S., Fennrich, S., Fischer, M., Gommer, M., Halder, M., Haslov, K., Jahnke, M., Montag-Lessing, T., Poole, S., Schechtman, L., Wendel, A., & Werner-Felmayer, G. (2001). Novel Pyrogen Tests Based on the Human Fever Reaction: The Report and Recommendations of ECVAM Workshop 43. ATLA, 29, 99-123.
Hartung, T., & Wendel, A. (1995). Detection of Pyrogens using human whole blood. ALTEX, 12(2), 353-359. https://pubmed.ncbi.nlm.nih.gov/11178418/
Hartung, T., & Wendel, A. (1996). Detection of pyrogens using human whole blood. In Vitro and Molecular Toxicology: Journal of Basic and Applied Research, 9(4), 353-359.
Kang, S., Lee, T. A., Ra, E. A., Lee, E., Choi, H. J., Lee, S., & Park, B. (2014). Differential control of interleukin-6 mRNA levels by cellular distribution of YB-1. PLoS ONE, 9(11). https://doi.org/10.1371/journal.pone.0112754
Mäkelä, S. M., Strengell, M., Pietilä, T. E., Österlund, P., &Julkunen, I. (2009). Multiple signaling pathways contribute to synergistic TLR ligand-dependent cytokine gene expression in human monocyte-derived macrophages and dendritic cells . Journal of Leukocyte Biology, 85, 664-672.
Moltz, H. (1993). Fever: causes and consequences. Neuroscience and Biobehavioral Reviews, 17(3), 237-269. https://doi.org/10.1016/S0149-7634(05)80009-0
Naik, E., & Dixit, V. M. (2011). Mitochondrial reactive oxygen species drive proinflammatory cytokine production. In Journal of Experimental Medicine (Vol. 208, Issue 3, pp. 417-420). https://doi.org/10.1084/jem.20110367
Peterbauer, A., Werner, E. R., & Werner-Felmayer, G. (2000). Weiterentwicklung eines Zellkulturmodells zum Nachweis bakterieller Pyrogene. 455-455. https://doi.org/10.1007/978-3-7091-6760-1_98
Pool, E. J., Johaar, G., James, S., Petersen, I., & Bouic, P. (1998). The detection of pyrogens in blood products using an ex vivo whole blood culture assay. Journal of Immunoassay, 19(2-3), 95-111. https://doi.org/10.1080/01971529808005475
TAKTAK, Y. S., SELKIRK, S., BRISTOW, A. F., CARPENTER, A., BALL, C., RAFFERTY, B., & POOLE, S. (1991). Assay of Pyrogens by Interleukin-6 Release from Monocytic Cell Lines. Journal of Pharmacy and Pharmacology: An International Journal of Pharmaceutical Science, 43(8), 578. https://doi.org/10.1111/j.2042-7158.1991.tb03540.x
Tilders, F. J. H., DeRuk, R. H., van Dam, A. M., Vincent, V. A. M., Schotanus, K., & Persoons, J. H. A. (1994). Activation of the hypothalamus-pituitary-adrenal axis by bacterial endotoxins: routes and intermediate signals. Psychoneuroendocrinology, 19(2), 209-232. https://doi.org/10.1016/0306-4530(94)90010-8
Tsuchiya, S., Yamabe, M., Yamaguchi, Y., Kobayashi, Y., Konno, T., & Tada, K. (1980). Establishment and characterization of a human acute monocytic leukemia cell line (THP-1). International Journal of Cancer, 26(2), 171-176. https://doi.org/10.1002/IJC.2910260208
Werner-Felmayer, G., Baier-Bitterlich, G., Fuchs, D., Hausen, A., Murr, C., Reibnegger, G., Werner, E. R., & Wachter, H. (1995). Detection of bacterial pyrogens on the basis of their effects on gamma interferon-mediated formation of neopterin or nitrite in cultured monocyte cell lines. Clinical and Diagnostic Laboratory Immunology, 2(3), 307-313. https://doi.org/10.1128/CDLI.2.3.307-313.1995
Zeisberger, E., & Roth, J. (1993). Neurobiological concepts of fever generation and suppression. Neuropsychobiology, 28(1-2), 106-109. https://doi.org/10.1159/000119010
Ziegler-Heitbroc, H. W. L., Thiel, E., Futterer, A., Herzog, V., Wirtz, A., & Riethmüller, G. (1988). Establishment of a human cell line (Mono Mac 6) with characteristics of mature monocytes. International Journal of Cancer, 41(3), 456-461. https://doi.org/10.1002/IJC.2910410324

## Claims

1. A method for the detection of pyrogens in a biological sample, in which immune cells are brought into contact with the sample, which may contain pyrogens, whereby the expression of immunomodulatory mediators takes place, **characterized in that** after lysis of the immune cells the nucleic acids of the immune cells are extracted and the expression of immunomodulatory mediators is detected by means of PCR.

2. A method according to claim 1, **characterized in that** the immune cells are cells which express Toll-like receptors (TLRs) or can be induced to express TLRs.

3. A method according to claim 1 or 2, **characterized in that** cultivated monocytes/macrophages, preferably the monocytic cell line THP-1 or THP-1 macrophages, serve as immune cells.

4. A method according to any one of claims 1 to 3, **characterized in that** the immunomodulatory mediators are pro-inflammatory mediators, preferably cytokines or chemokines, in particular IL-1β, TNFα or CXCL8/IL-8

5. A method according to any one of claims 1 to 4, **characterized in that** the detection of expression by measuring the amplification of messenger RNA (mRNA) uses quantitative real-time PCR (qPCR) or digital PCR (dPCR).

6. A method according to one of claims 1 to 5, **characterized in that** at least one of three different cytokines/chemokines, preferably IL-1β, TNFα or CXCL8/IL-8, are detected in parallel.

7. A method according to any one of claims 1 to 6, **characterized in that** a housekeeping gene, preferably housekeeping gene TATA-binding protein (TBP), is added to control data accuracy.

8. A method according to any one of claims 1 to 7, **characterized in that** additionally either a fourth cytokine IL-6 or an internal extraction control RNA (IK_RNA) is added.

9. A method according to any one of claims 3 to 7, comprising following steps:
a) seeding of cryopreserved monocytes/macrophages in cell culture plates with preferably 96 wells
b) combination of the sample with the monocyte/macrophage system capable of responding to the sample with the production of certain proinflammatory cytokines
c) addition of a lysis buffer and transfer of the cell culture plate containing monocytes/macrophages into an automated extraction system, whereby the released nucleic acids are bound to a solid phase
d) washing and purification of the nucleic acids bound to the solid phase
e) DNA amplification of the nucleic acids and performance of PCR to detect gene expression of the immunomodulatory mediators.

10. A method according to claim 9, wherein a primer/probe system for three cytokines (IL-1β, TNFα, IL-8) or for four cytokines (IL-1β, TNFα, IL-8, IL-6) as well as primers/probes for a housekeeping gene TBP are added to the step of DNA amplification of the nucleic acids.

11. A method according to claim 9 or 10, wherein an elevated level of at least one of the fever-relevant cytokines (IL-1β, TNFα, IL-8 or IL-6) indicates the presence of a pyrogen in the sample.

12. A detection kit for carrying out the method according to any one of claims 1 to 11, comprising:
a) cultured monocytes/macrophages
b) cell culture plates with preferably 96 wells
c) at least one lysis buffer and buffer for binding the nucleic acids released by lysis
d) a solid phase, preferably magnetic beads
e) PCR reagents for amplification of DNA, a primer/probe system containing primers/probes for at least three cytokines/chemokines.

13. Test kit according to claim 12, additionally comprising:
f) a housekeeping gene, preferably housekeeping gene TATA-binding protein (TBP)
g) a fourth cytokine IL-6 or an internal extraction control RNA (IK_RNA)
h) primers/probes for the housekeeping gene TBP
i) primers/probes for one fourth cytokine.

14. Using the method according to any one of claims 1 to 11 or the test kit according to claim 12 or 13 to detect endotoxin and non-endotoxin contamination in
a) pharmaceutical products, preferably in parenteral medical products, dialytes, vaccines or intravenous solutions; or
b) non-medicinal fluid that are not applied orally or get in contact with bodily fluids
c) medical products
d) nutrients.

15. Use of the method according to any one of claims 1 to 11 or the test kit according to claim 12 or 13 for the detection of IL-6 gene expression.
